# EUROPEAN PATENT APPLICATION

(11) **EP 4 527 462 A2**
(43) Date of publication of application: **26.03.2025**
(21) Application number: 25155022.4
(22) Date of filing: 06.04.2021
(51) Int. Cl.: A61P 31/16

(54) **USE OF AN ARSENIC COMPOUND FOR TREATING A SHORT OR LONG CYTOKINE STORM IN VARIOUS AUTOIMMUNE / INFLAMMATORY DISEASES IN HUMANS OR ANIMALS**

(30) Priority: 03.04.2020 EP 20168111; 22.10.2020 EP 20306261
(62) Divisional of application: 21715929.2
(71) Applicant: Medsenic, 67100 Strasbourg (FR)
(72) Inventor: RIEGER, François, 1203 GENEVE (CH); RIEGER, Simon, 34250 LA GRANDE MOTTE (FR)
(74) Representative: Santarelli

(57) **Abstract**

The present disclosure relates to the use of an arsenic compound for treating a cytokine storm in a patient in need thereof.

## Description

### FIELD OF THE INVENTION

The present invention pertains to the field of therapy. More precisely, the invention provides a new therapeutic approach for treating uncontrolled and excessive release of pro-inflammatory cytokines, known as hypercytokinemia or cytokine storm.

### BACKGROUND

### Cytokine storm

An hypercytokenemia, or cytokine storm, is observed during certain severe reactions to a variety of microbial infections, such as lung infections or during the course of flares of autoimmune diseases or other diseases with a marked inflammatory component. It has been observed that local lung infection and inflammation too often get into the general blood circulation, and may end up into systemic infection and sepsis. Systemic sepsis - an exaggerated proinflammatory cytokine release - is generally accompanied by persistent hypotension, hyper- or hypothermia, leukocytosis or leukopenia, and thrombocytopenia (Levy et al., 2003), and often leads to death. It is widely observed that viral, bacterial, and/or fungal pulmonary infections may all cause a sepsis syndrome. Nowadays, these infectious agents can be biochemically characterized through adequate molecular testing. It is known by specialists of the immune system (in particular specialists of innate immunity) that, in many instances, persistent tissue damage - specifically in the lungs affected by interstitial diseases - is associated with a cytokine storm of variable duration. In such circumstances, clinical manifestations seem to be highly reminiscent of a sepsis syndrome.

It is worth mentioning here that different effects of a cytokine storm, of graded severity and variable duration, have been observed in various categories of individuals with a number of diseases, either acute or chronic. This is indeed well documented in the lungs, an organ particularly reactive to diverse noxious stimuli (Wurfel MM, et al. 2005), related to identified infections or other types of sustained injury including environment causes, leading to fibrosis, with the obvious involvement and overproduction of cytokines or growth factors, especially in the lung epithelium.

There even have been tentative explanations linking cytokine levels and variable sensitivities to several microbial agents, as exemplified in malaria or other microbial diseases (Mockenhaupt FP, et al. 2006a; Mockenhaupt FP, et al. 2006b). They consistently display a marked cytokine storm event, although of different severity or duration.

One very important example of viral infection provoking a cytokine storm is the outbreak of coronavirus disease 2019 (COVID-19) caused by the severe acute respiratory syndrome coronavirus 2 (SARS-CoV-2/2019-nCoV), resulting in a huge number of infected and dead people calling for an urgent need of effective, available, and affordable drugs to control and diminish the epidemic.

SARS-CoV entry is essentially mediated through the interaction of the ACE2 receptor and the viral spike protein. Time-of-addition experiments of various drugs have tried not only to inhibit, *in vitro,* the entry step, as well as the post-entry intracellular infectious stages of SARS-CoV-2 involving early endosomes (EEs) or endolysosomes (Els) but also the consequences of the synthesis and release of viral biochemical components of the virus on the immune system defenses in the infected individual, either cellular (specialized T cells, B cells, dendritic cells, monocytes and macrophages of the immune system) or molecular (signaling cytokines and chemokines) components.

Chinese clinical investigations first reported that high concentrations of cytokines were detected in the plasma of critically ill patients infected with SARS-CoV-2, suggesting that a cytokine overproduction or storm was associated with disease severity (Huang, C. et al., 2020).

Thus, there is an absolute need for an anti-inflammatory agent that could significantly decrease the production of pro-inflammatory factors and specifically cytokines, in critically ill patients.

In direct relation to the coronavirus infection, it is of high interest to mention an early work of French researchers in an *in vivo* animal model of an innate immune response to a coronavirus previously found in a porcine species (B. Charley, Bull. Acad. Vét. France 6 2003 -Vol. 156 pp 31-36 - Supplement to N° 3). In this study, the authors observed a very high production of interferon alpha in response to a viral infection of piglets by the porcine transmissible gastroenteritis virus (TGEV). Not only a stimulated production of interferon alpha was systematically demonstrated, but the cells responsible for the strong interferon alpha release were identified: plasmacytoid dendritic cells (pDCs) and monocytes/macrophages. These types of cells are found in respiratory viruses infected humans, most frequently in the respiratory tract, nasal mucosa and lungs. The authors even suggest that the levels of blood interferon alpha could be used as a measure of the intensity and severity of the disease.

In addition, it is now known that high and/or sustained level of interferon alpha has deleterious consequences, leading to too strong immune reactions, in turn responsible for pathologies involving inflammation and autoimmunity (Crow and Rodero, 2016).

More generally, one can anticipate that decreasing such specific cytokines during a given cytokine overproduction event or storm would be a way to decrease the deleterious damage brought to the organs affected by the excessive production of cytokines, such as the respiratory system of mammalian organisms infected with severe acute respiratory syndrome viruses. This is true not only for the Covid 19 critically ill patients, but also in other infectious viral diseases, for example those caused by SARS or MERS recent epidemics, as well as influenza viruses. More broadly, this conclusion can be extended to other conditions such as autoimmune diseases, inflammatory diseases and neurodegenerative diseases with an inflammatory component, as listed below.

In short, there may be numerous disease states which could be worsened or even be mainly explained by a short or long lasting cytokine storm, not only triggered by initial infectious or related causes, but even related to non infectious causes in humans and other mammalian species, characterized by an innate immune system often mobilized in the early steps of various diseases, such as those listed below.

A non-exhaustive list of some of such ailments includes:
- Autoimmune diseases, with exacerbations of the immune system functions,
- Less well defined diseases with primary or secondary exacerbations of the immune system in organs of critical physiological function, such as the lungs in systemic sclerosis, and related diseases, such as Graft versus Host disease, or less defined interstitial lung diseases (idiopathic interstitial diseases)
- Primary inflammatory diseases with activation of the innate immune system functions
- Infectious diseases, with initial bacterial, viral or fungal primary attacks
- Neurodegenerative diseases with a strong autoimmune or inflammatory component, such as multiple sclerosis, Parkinson's and Alzheimer's diseases, bipolar disorder and schizophrenia and their related pathological entities.

Molecular components of the cytokine storm include many with direct inflammatory properties and a few others with anti-inflammatory properties (often acting like direct stimulators or inhibitors of the sustained cytokine storm).

A non-exhaustive list of such cytokines (Akdis et al. 2016) includes:
- IL18, IL18BP, IL1A, IL1B, IL1F10, IL1F3/IL1RA, IL1F5, IL1F6, IL1F7, IL1F8, IL1RL2, IL1F9, IL33
- IL-1 Receptors: IL18R1, IL18RAP, IL1R1, IL1R2, IL1R3, IL1R8, IL1R9, IL1RL1, SIGIRR
- TNF family: BAFF, 4-1BBL, TNFSF8, CD40LG, CD70, CD95L/CD178, EDA-A1, TNFSF14, LTA/TNFB, LTB, TNFa, TNFSF10, TNFSF11, TNFSF12, TNFSF13, TNFSF15, TNFSF4
- TNF Receptor: 4-1BB, BAFFR, TNFRSF7, CD40, CD95, DcR3, TNFRSF21, EDA2R, EDAR, PGLYRP1, TNFRSF19L, TNFR1, TNFR2, TNFRSF11A, TNFRSF11B, TNFRSF12A, TNFRSF13B, TNFRSF14, TNFRSF17, TNFRSF18, TNFRSF19, TNFRSF25, LTBR, TNFRSF4, TNFRSF8, TRAILR1, TRAILR2, TRAILR3, TRAILR4
- Interferon (IFN): IFNA1, IFNA10, IFNA13, IFNA14, IFNA2, IFNA4, IFNA7, IFNB1, IFNE, IFNG, IFNZ, IFNA8, IFNA5/IFNaG, IFNw/IFNW1,
- IFN Receptor: IFNAR1, IFNAR2, IFNGR1, IFNGR2
- IL6 Family: CLCF1, CNTF, IL11, IL31, IL6, Leptin, LIF, OSM
- IL6 Receptor: CNTFR, IL11RA, IL6R, LEPR, LIFR, OSMR, IL31RA,
- IL10 Family: IL10, IL19, IL20, IL22, IL24, IL28B, IL28A, IL29
- IL10 Family Receptor: IL10RA, IL10RB, IL20RA, IL20RB, IL22RA2, IL22R
- TGF beta Family: TGF-beta 1/TGFB1, TGF-beta 2/TGFB2, TGF-beta 3/TGFB3,
- TGF beta Family Receptor: ALK-7, ATF2, CD105/ENG, TGFBR1, TGFBR2, TGFBR3
- Chemokine: CCL1/TCA3, CCL11, CCL12/MCP-5, CCL13/MCP-4, CCL14, CCL15, CCL16, CCL17/TARC, CCL18, CCL19, CCL2/MCP-1, CCL20, CCL21, CCL22/MDC, CCL23, CCL24, CCL25, CCL26, CCL27, CCL28, CCL3, CCL3L3, CCL4, CCL4L1/LAG-1, CCL5, CCL6, CCL7, CCL8, CCL9, CX3CL1, CXCL1, CXCL10, CXCL11, CXCL12, CXCL13, CXCL14, CXCL15, CXCL16, CXCL17, CXCL2/MIP-2, CXCL3, CXCL4, CXCL5, CXCL6, CXCL7/Ppbp, CXCL9, IL8/CXCL8, XCL1, XCL2, FAM19A1, FAM19A2, FAM19A3, FAM19A4, FAM19A5
- Chemokine Receptor: CCR1, CCR2, CCR3, CCR4, CCR5, CCR6, CCR7, CCR8, CCRL1, CXCR3, CXCR4, CXCR5, CXCR6, CXCR7, CXCR1, CXCR2
- S100A8 and S100A9.

### Arsenic

Arsenic compounds have been widely used by traditional medicine in many parts of the world, for the treatment of various diseases such as syphilis, psoriasis, or rheumatic arthritis, for centuries. Many different arsenic preparations including various arsenic species have been developed and used during the long history of these agents.

Some arsenical species are notably toxic and even carcinogenic on the long run, with side effects such as cirrhosis of the liver, idiopathic portal hypertension, urinary bladder cancer, and skin cancers. Recently however, several arsenic compounds have been revisited and carefully formulated to treat different categories of diseases, and prominently cancer.

In particular, Arsenic Trioxide (As₂O₃, also noted "ATO" in the present text) happens to be one of the most effective novel anticancer ("antineoplastic" or "cytotoxic") agent. ATO has been approved by the US FDA and EU EMA for the treatment of acute promyelocytic leukemia (APL) resistant to "first line" agents, namely all-trans retinoic acid (ATRA). It has been shown that arsenic trioxide induces dividing cancer cells to undergo apoptosis.

Arsenic Trioxide is also known or currently investigated as an agent against other diseases, namely auto-immune diseases.

Bobé et al. (Blood, 108, 13, p3967 - 3975, 2006) investigated the effects of arsenic trioxide in a mouse model of systemic lupus erythematosus. As₂O₃ significantly prolonged survival of MRL//*pr* mice by preventing young mice from developing the disease and quasi-totally reversing established disease in older animals. These authors suggested that this compound might be useful in the treatment of human systemic lupus erythematosus and this is indeed what has been established (Hamidou et al, 2021).

Among other contributions, Kavian et al. (J Immunol. 2012;188(10):5142-9) reported the successful use of ATO in murine sclerodermatous GvHD.

More recently, Maier et al. (J. Immunol. 2014 Jan 15;192(2):763-70) demonstrated that arsenical compounds, including ATO, are potent inhibitors of caspase-1 and the innate immune response (namely mediated by Interleukin 1-β) and thus may have potential for the treatment of inflammatory components of autoimmune disorders. Furthermore, Li et al. showed that arsenic trioxide improves Treg and T17 balance in rheumatoid arthritis patients, thus being a potential useful immune modulator (Int. Immunopharmacol., 2019, Arsenic trioxide improves Treg and Th 17 balance by modulating STAT3 in treatment-naïve rheumatoid arthritis patients*).*

To explain the recent observations of the As beneficial action on the immune system, a new mechanism of action was proposed. Indeed, it becomes clear that one of the major consequences of the exposure of a number of cell types to As both *in vitro* (cell lines or primary cultures, normal or diseased) and *in vivo* (animal models for autoimmune diseases) is the activation or inhibition of specific cell signaling pathways and cell death, with the frequent stimulation of various proapoptotic programs.

### SUMMARY

The present invention is based by the demonstration, by the inventors, that arsenic compounds can inhibit a wide variety of specific cytokines involved in cytokine storms of different origin.

The present invention thus pertains to the use of a composition comprising an arsenic compound for preventing or alleviating a cytokine overproduction or cytokine storm, either acute or prolonged.

In particular, the present invention relates to a method for treating a SARSr-CoV infection in a patient in need thereof, comprising the step of administering a therapeutically effective dose of an arsenic compound to said patient.

The present invention also relates to a method for treating an autoimmune disease like RR (relapsing Remitting) or (Progressive,P) or secondary Progressive (SP) Multiple Sclerosis, in a patient in need thereof, comprising the step of administering a therapeutically effective dose of an arsenic compound to said patient.

Another aspect of the present invention is a method for treating a neurodegenerative disease with an autoimmune or inflammatory component, like Parkinson's, Alzheimer's diseases and their related diseases, in a patient in need thereof, comprising the step of administering a therapeutically effective dose of an arsenic compound to said patient.

The present invention also pertains to a method for treating a mental disease with an autoimmune or inflammatory component, like bipolar disorder or Schizophrenia or depression, in a patient in need thereof, comprising the step of administering a therapeutically effective dose of an arsenic compound to said patient.

### LEGENDS TO THE FIGURES:

**Figure 1****: IFNα production after TLR-9 stimulation by dinucleotides (DNA, RNA stimulations) and inhibition by different concentrations of ATO.** Mean ± SEM, n=3 donors (each condition performed in triplicates)
**Figure 2****: IL-6 production after TLR-9 stimulation by dinucleotides (DNA, RNA stimulations) and inhibition by different concentrations of ATO.**
**Figure 3****: IL-1β production after TLR-9 stimulation by dinucleotides (DNA, RNA stimulations) and inhibition by different concentrations of ATO.** Mean ± SEM, n=3 donors (each condition performed in triplicates)
**Figure 4****: Mean production of TNFα in basal conditions and after stimulation with the different test items in absence or presence of ATO.** Mean ± SEM, n=3 donors (each condition performed in triplicates). *p<0.05, ***p<0.001 in comparison to the respective control group.
**Figure 5****: Relative production of TNFα after stimulation of TLR4 with 100 nM of S Spike protein.** Mean ± SEM, n=3 donors (each condition performed in triplicates). ***p<0.001 in comparison to S Spike 100 nM.
**Figure 6****: Mean production of IL-1β in basal conditions.** Mean ± SEM, n=3 donors (each condition performed in triplicates). ***p<0.001 in comparison to the respective control group
**Figure 7****: Relative production of IL-1β after stimulation with 100 nM of S Spike protein.** Mean ± SEM, n=3 donors (each condition performed in triplicates). ***p<0.001 in comparison to S Spike 100 nM.
**Figure 8****: Mean production of IL-6 in basal conditions and after stimulation with the different test items in absence or presence of ATO.** Mean ± SEM, n=3 donors (each condition performed in triplicates). *p<0.05, ***p<0.001 in comparison to the respective control group.
**Figure 9****: Relative production of IL-6 after stimulation of TLR4 with 100 nM of S Spike protein.** Mean ± SEM, n=3 donors (each condition performed in triplicates). ***p<0.001 in comparison to S Spike 100 nM.
**Figure 10****: Mean production of IL-8 in basal conditions and after stimulation with the different test items in absence or presence of ATO.** Mean ± SEM, n=3 donors (each condition performed in triplicates). ***p<0.001 in comparison to the respective control group.
**Figure 11****: Relative production of IL-8 after stimulation with 100 nM of S Spike protein.** Mean ± SEM, n=3 donors (each condition performed in triplicates). **p<0.01 in comparison to S Spike 100 nM
**Figure 12****: Relative production of IL-1β after stimulation with protein PX.** Mean ± SEM, n=3 donors (each condition performed in triplicates).

### DETAILED DESCRIPTION

The experimental part which follows describes new, original and innovative results from studies aimed at inhibiting part or all of a given cytokine overproduction or storm, in order to treat a sepsis syndrome or more generally a cytokine overproduction or storm originating from any infectious event or disease state involving increased or sustained overproduction of cytokines of proinflammatory nature, such as IL-1 beta, TNF alpha or IL6 among the most prominent ones.

In the experimental settings described below, using fresh human blood cells (PBMCs) stimulated by nucleotides or proteins, both involved in the very primary processes of microbial infections, the inventors observed that ATO, known to be directly interfering with certain important cell pathways, can also inhibit the beginning and/or sustained cytokines overproduction.

This inhibition of specific cytokines involved in cytokine overproduction has been demonstrated in three different experimental systems:
1/ Human PBMCs from healthy human donors stimulated *in vitro* by dinucleotides originating from diverse foreign or even self DNAs or RNAs, known to activate a cascade of cell reactions originating in the molecular activation of Toll like 7/9 receptors on the extracellular membranes of circulating (specialized) hematopoietic cells in the blood and resulting in high production and release of key cytokines by PBMCs, including interferon species, various interleukins and others, mostly involved in inflammation processes in response to microbial invasions.
2/ Human PBMCs from healthy human donors stimulated *in vitro* by the SARS-CoV-2 Spike (S) glycoprotein, able to activate TLR4, leading to a high release of key cytokines, including tumor necrosis factors, interleukins and others generally involved in inflammation processes in response to microbial invasions.
3/ Human PBMCs from healthy human donors stimulated *in vitro* by HERV W Env Protein (designated Protein PX) resulting in high production and release of cytokines, as best exemplified by IL-1β.

The present invention is based on our original observations showing that a cytokine production can be controlled by an arsenic salt, demonstrating that arsenic compounds can be used in therapeutic interventions to limit or stop cytokine overproductions or storms in humans and animals (with special emphasis on the mammalian species).

According to a first aspect, the present invention thus pertains to the use of a composition comprising an arsenic compound, as a medicament for preventing and/or alleviating a cytokine storm.

As used herein, "preventing", indicates an approach for preventing, inhibiting, or reducing the likelihood of the occurrence of a cytokine storm.

As used herein, "alleviating" or "treating" and similar words mean reducing the intensity, effects, symptoms and/or burden of a cytokine storm.

Arsenic trioxide (ATO), already well known in the pharmacopeia, is the leading active molecule of the family of arsenic salts.

Other arsenic salts which can be used according to the invention are described in the following table:

**Table 1: arsenic compounds which can be used according to the present invention.**

| Formula | Name | CAS number |
|---|---|---|
| As | Arsenic | 7440-38-2 |
| AsBrO | Arsenic Oxybromide | 82868-10-8 |
| AsBr₃ | Arsenic tribromide | 7784-33-0 |
| C₃H₉As | Trimethylarsin | 593-88-4 |
| AsCl₃ | Arsenic trichloride | 7784-34-1 |
| AsCl₃O | Arsenic oxychloride | 60646-36-8 |
| AsCl₅ | Arsenic pentachloride | 22441-45-8 |
| AsF₃ | Arsenic trifluoride | 7784-35-2 |
| AsF₅ | Arsenic pentafluoride | 7784-36-3 |
| AsH₃ | Arsenic trihydride | 7784-42-1 |
| AsI₂ | Arsenic diiodide | 13770-56-4 |
| AsI₃ | Arsenic triiodide | 7784-45-4 |
| AsO | Arsenic monoxide | 12005-99-1 |
| AsO₂ | Arsenic dioxide | 12255-12-8 |
| AsP | Arsenic monophosphide | 12255-33-3 |
| AsP₃ | Arsenic phosphide | 12511-95-4 |
| AsSe₄ | Arsenic tetra selenide | 12006-06-3 |
| As₂H₄ | diArsenic tetrahydride | 15942-63-9 |
| As₂I₄ | diArsenic diiodide | 13770-56-4 |
| As₂O₃ | arsenic trioxide | 1327-53-3 |
| As₂O₅ | Arsenic pentoxide | 1303-28-2 |
| As₂P₂ | Arsenic diphosphide | 12512-03-7 |
| As₂S₃ | Arsenic disulphide | 1303-33-9 |
| As₂S₄ | Arsenic tetrasulphide | 1303-32-8 |
| As₂S₅ | Arsenic pentasulphide | 1303-34-0 |
| As₂Se | Arsenic hemiselenide | 1303-35-1 |
| As₂Se₃ | Arsenic triselenide | 1303-36-2 |
| As₂Se₅ | Arsenic pentaselenide | 1303-37-3 |
| As₂Te₃ | Arsenic tritelluride | 12044-54-1 |
| As₃O₄ | Arsenic tetraoxide | 83527-53-1 |
| As₃P | Triarsenic phosphide | 12512-11-7 |
| As₄S₄ | Realgar | 12279-90-2 |
| As₄S₆ | Orpiment | 12255-89-9 |

Among the arsenic salts listed in table 1, As₂O₃, AsI₃, As₂O₅, As₄O₆, As₂S₂, As₂S₃, As₂S₅ and As₄S₄ are particularly appropriate active ingredients for treating or preventing a cytokine storm. According to the invention, these compounds can be used alone or in a mixture of two or more of these salts (e.g., As₂O₃ + As₂O₅).

According to a preferred embodiment of the invention, arsenic trioxide and/or arsenic triiodide are used as active ingredient(s).

According to a particular embodiment, the composition can comprise, in addition to the arsenic compound(s), a metal ion selected from the group consisting of Cu2+, Au2+, Fe2+, Zn2+, Mn2+, Mg2+ and mixtures thereof, to potentiate the effects of the arsenic compounds, as disclosed in PCT/EP2020/064189, filed on May 20, 2020.

In the frame of the present invention, the skilled person can chose any appropriate means for administering the arsenic compound. In particular, the skilled person can adapt the pharmaceutical form, method and route of administration to the patient's condition, including the location of the infection at the origin of the (possible) cytokine storm, the patient's ability to swallow a capsule, *etc.*

According to a particular embodiment, the arsenic compound is administered intravenously.

According to another particular embodiment, the arsenic compound is administered as an aerosol spray.

According to another particular embodiment, the arsenic compound is administered orally.

According to another particular embodiment, the arsenic compound is administered topically.

The arsenic compound can be administered via specific preparations involving nanoparticles of different compositions, such as pre-packaged preparations for topical administration or oral formulations in the liquid or solid form or liposomal-like nanoparticles. Depending on the indication, the arsenic compound can also advantageously be included in formulations including synergic mixtures of molecules, such as corticosteroids (dexamethasone for example), colchicine, propolis or bee venom, extracted immune system active components, monoclonal antibodies directed towards any relevant proteic component of the immune system and more generally any compound with identified action on any component of the immune system.

According to a particular embodiment, the composition used according to the invention reduces the severity of the cytokine overproduction or storm.

According to a more specific embodiment, the composition used according to the invention reduces IFNα, INFγ, TNFα, IL-6, IL-1β, IL-8, GM-CSF, IL17, IL23 and/or IL-10 production by peripheral blood mononuclear cells (PBMCs) or immune cells resident in the lymphoid organs or CNS microglial cells, thereby preventing and/or alleviating an overproduction of cytokines or a cytokine storm of any intensity or duration.

According to another of its aspects, the present invention relates to the use of a composition as described above, for treating a condition which can possibly provoke an overproduction of cytokines or a cytokine storm. Depending on the condition (e.g., infectious agent) and host parameters, the composition of the invention can be used alone or in combination with other active ingredients, such as antipyretics or other modulators of specific components of the innate immune system, anti-inflammatory agents, antibiotics and antiviral agents.

According to a particular embodiment of the invention, the composition is used for treating an infectious disease caused by a pathogen selected amongst *betaviridae* such as severe acute respiratory syndrome-related coronavirus (SARSr-CoV), Middle East respiratory syndrome-related coronavirus (MERS-CoV) and porcine transmissible gastroenteritis virus (TGEV), *alphaviridae* such as *influenza* and chikungunya, hantavirus, Marburg and Ebola viruses, Lassa and Junin viruses, dengue viruses, a *Plasmodium* parasite (e.g., *Plasmodium falciparum, Plasmodium vivax, Plasmodium ovale, Plasmodium malariae* and *Plasmodium knowlesi)* and any bacteria, especially involved in bacterial sepsis.

According to a more particular embodiment of the invention, the composition is used for treating a critically ill patient infected by a SARSr-CoV virus or its variants, for example a patient who suffers from CoViD-19.

According to a particular aspect, the present invention pertains to a method for treating a SARSr-CoV infection in a subject in need thereof, comprising the step of administering a therapeutically effective dose of an arsenic compound to said subject.

A "therapeutically effective amount" of an arsenic-containing compound may vary according to factors such as the nature of the arsenic salt and the composition in which it is formulated (e.g., the possible combination with a metal ion), disease state, age, sex, and weight of the individual, *etc.* A therapeutically effective amount is also one in which any toxic or detrimental effects of the agent are outweighed by the therapeutically beneficial effects. The term "therapeutically effective amount" includes an amount that is effective to treat a subject and/or prevent the onset of a cytokine storm in this subject.

Any of the arsenic compounds mentioned above can be used to perform the method according to the invention. In particular, the arsenic compound can advantageously be selected from the group consisting of As₂O₃, AsI₃, As₄O₆, As₂O₅, As₂S₂, As₂S₃, As₂S₅, As₄S₄ and mixtures thereof, preferably arsenic trioxide and/or arsenic triiodide.

The invention disclosed herein can be used to treat a human or an animal. The doses indicated below are those calculated for a human individual.

According to a particular embodiment of the above method, arsenic trioxide (or the like) is administered to a subject in need thereof at a daily dose of 0.01 to 5 mg/kg of bodyweight.

According to another particular embodiment of the method, arsenic trioxide is administered to a subject in need thereof at a daily dose of 0.05 to 0.5 mg/kg of bodyweight.

According to another particular embodiment of the method, arsenic trioxide is administered to a subject in need thereof at a daily dose of 0.05 to 0.30 mg/kg of bodyweight.

According to another particular embodiment of the method, arsenic trioxide is administered to a subject in need thereof at a daily dose of 0.10 to 0.30 mg/kg, for example 0.10 to 0.20 mg/kg of bodyweight.

According to another particular embodiment of the method, arsenic trioxide is administered to a subject in need thereof at a daily dose of 0.075 to 0.30 mg/kg, preferably around 0.15 mg/kg of bodyweight.

According to yet another particular embodiment of the method, the arsenic compound is administered to a subject in need thereof in combination with a metal ion selected from the group consisting of Cu²⁺, Au²⁺, Fe²⁺, Zn²⁺, Mn²⁺, Mg²⁺ and mixtures thereof.

More particularly, when arsenic trioxide is administered to a subject in need thereof in combination with a metal ion as above described, it is preferably formulated so that one daily dose of ATO is from 0.01 to 0.15 mg/kg/day.

In the frame of the present invention, a "subject in need of" a treatment can designate any person infected by a SARSr-CoV (e.g., SARS-Cov2) or any other condition likely to provoke an overproduction of cytokines or a cytokine storm.

Non-exhaustive examples of vulnerable populations for SARSr-CoV, who can be considered as in need of a treatment according to the present invention, include:
- older adults (> 60, >65, >70, >75, >80, >85, >90 years);
- people of any age with chronic medical conditions (for example, interstitial lung diseases, cardiovascular diseases, vascularites of diverse origin, high blood pressure, diabetes type 1 or 2, kidney diseases, liver disease, Multiple Sclerosis, Parkinson's and Alzheimer's diseases, stroke or dementia, autoimmune and idiopathic schizophrenia, bipolar disorders with possible involvement of endogenous retroviral proteins);

- people of any age who are immunocompromised, including those with an underlying medical condition (for example, cancer) or taking medications which lower the immune system (for example, chemotherapy); and
- people living with obesity (BMI of 40 or higher) or any predisposing condition - such as inflammaging - for an exaggerated production of cytokines.

According to a particular embodiment, the method according to the invention is for treating a SARSr-CoV infection in a subject who suffers from CoViD-19 or is at high risk of a contamination, such as contact cases.

According to another particular embodiment, the method according to the invention is for treating a SARSr-CoV infection in subject who suffers from respiratory impairment.

More generally, the method according to the invention can advantageously be used for treating a patient suspected or confirmed to develop or have an infection by a SARSr-CoV or any other exogenous virus likely to provoke a cytokine storm.

As illustrated in example 4 below, the method according to the invention can advantageously be used for treating a patient suspected or confirmed to develop or have an inflammatory reaction linked to a human endogenous retroviral (HERV) component. Human endogenous retroviruses (HERVs), sometimes called fossil viruses, are the remnants of ancient retroviral infections. Around 8 per cent of the human genome is thought to comprise HERVs. In some cases, HERV components are able to be synthetized and released by a cell upon the right stimulus, for example following an infection by another virus.

According to a particular aspect of the invention, a patient suspected or confirmed to develop or have an infection by a SARSr-CoV or any other exogenous virus or endogenous virus likely to provoke a cytokine storm is treated as follows:
(i) at first symptoms: using any standard of care for RNA type viral infections to start decreasing the viral load, like first hypothesized for hydroxychloroquine (200-600 mg/day), possibly combined with azythromycine (200 mg/day) and/or Zn+ (15 mg/day), or remdesivir or a cocktail of antibodies against the virus proteins and/or specific pathogenic cytokine(s), such as INFα, IL6 or the like.
(ii) upon worsening of clinical signs (including slight respiratory impairment), immediately measure, if possible, the level of one or several proinflammatory cytokines related to the given infection or condition;
(iii) if suspicion exists or if - optimally - the test of step (ii) shows that some cytokines levels of are up by at least three times their normal circulatory levels, initiate a treatment with a composition comprising an arsenic compound, as described above.

Other characteristics of the invention will also become apparent in the course of the description which follows of the biological *in vitro* assays which have been performed in the framework of the invention and which provide it with the required experimental support, without limiting its scope.

### Examples:

### Example 1: The cytokine storm induced by the stimulation of PBMCs by viral nucleotidic components specific for the Toll like 9 receptors is inhibited by arsenic salts (arsenic trioxide)

**Objective:** The objective of this study was to determine the immunomodulatory effects of arsenic trioxide (ATO) on fresh human Peripheral Blood Mononuclear Cells (hPBMCs) in basal condition and after TLR-9 stimulation of cytokines production.

**Methods:** PBMCs were isolated from blood of 3 donors (provided by EFS, Hauts-de-France-Normandie) and stimulated with TLR-9 agonist in presence or absence of ATO. Furthermore, PD0325901, an inhibitor of MEK1/2-ERK signaling pathway, was added at the condition of ATO 2.5 µM, to assess the implication of this signaling pathway in the ATO-triggered inhibition of IFNα production. Twenty-four hours after stimulation, supernatants were harvested for cytokines analysis (IFNα, IL-6 and IL-1β).

### Study design

**Table 2: study design (experiments related to TLR-9-stimulation)**

| **Group** | **Stimulation** | **Treatment** | **Number of wells** | **Time of incubation** |
|---|---|---|---|---|
| 1 | Non-stimulated: ODN2216 Neg Ctrl (ODN2243) 1µM | Vehicle | 3 wells | |
| 2 | | ATO 0.1 µM | 3 wells | |
| 3 | | ATO 0.25 µM | 3 wells | |
| 4 | | ATO 0.5 µM | 3 wells | |
| 5 | | ATO 1 µM | 3 wells | |
| 6 | | ATO 2.5 µM | 3 wells | |
| 7 | | PD0325901 1 µM + ATO 2.5 µM | 3 wells | |
| 8 | | Positive control - Dexamethasone 1 µM | 3 wells | 24h |
| 9 | TLR-9-stimulated: ODN2216 1µM | Vehicle | 3 wells | |
| 10 | | ATO 0.1µM | 3 wells | |
| 11 | | ATO 0.25 µM | 3 wells | |
| 12 | | ATO 0.5 µM | 3 wells | |
| 13 | | ATO 1 µM | 3 wells | |
| 14 | | ATO 2.5 µM | 3 wells | |
| 15 | | PD0325901 1 µM + ATO 2.5 µM | 3 wells | |
| 16 | | Positive control - Dexamethasone 1 µM | 3 wells | |

Twenty-four hours following incubation with TLR-9 stimulants and test items (ATO or vehicle or reference control), each well content was harvested in Eppendorf tubes and centrifuged at 300g for 8 minutes. Supernatants were collected and stored at -70°C for cytokines analysis, while cell pellets were stored at -70°C for further optional analysis (upon sponsor request).

### Results:

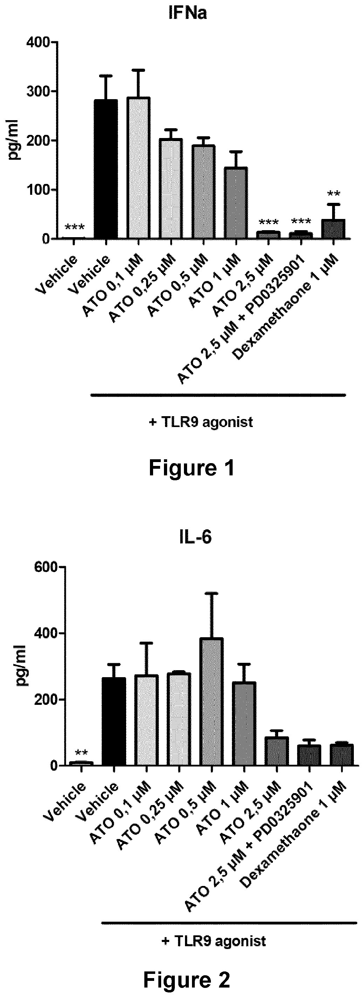

Figure 1 shows the production of IFNα after TLR-9 stimulation, in presence of different concentrations of ATO.

Figure 2 shows the production of IL-6 after TLR-9 stimulation, in presence of different concentrations of ATO.

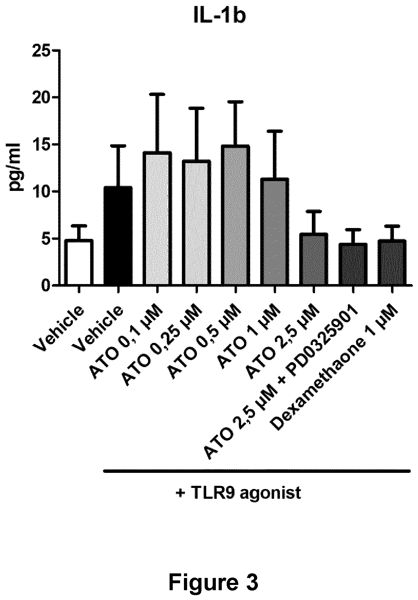

Figure 3 illustrates the production of IL-1β after TLR-9 stimulation in presence of different concentrations of ATO.

Interestingly, ATO modulated IL-6 and IL-1β production in TLR-9-stimulated hPBMCs in a bell-shape-like manner, since it slightly increased their production at low dose and inhibited it at the higher tested dose (2.5 µM ATO). Positive control dexamethasone strongly inhibited the production of both IFNα, IL-6 and IL-1β after TLR-9 stimulation. PD0325901 did not restore the production of IFNα after inhibition by ATO in TLR-9 stimulated cells.

ATO did not affect the production of IFNα and IL-1β in basal conditions (non-TRL-9-stimulated hPBMCs). However, the concentration of IL-6 in basal conditions was dependent on ATO concentration in a bi-phasic fashion, with an increase of IL-6 production for ATO doses up until 0.5 µM, followed by a decrease for ATO doses up to 2.5µM.

TLR-9 stimulation increased the production of IFNα, IL-6 and IL-1β from hPBMCs from all the 3 donors

**Conclusion:** After TLR-9 stimulation, ATO strongly inhibited the production of IFNα in a dose-dependent manner, as well as the production of IL-6 and IL-1β.

### Example 2: The cytokine storm stimulated by the S Spike glycoprotein is inhibited by arsenic salts (arsenic trioxide)

### Objective:

The aim of this study was to preliminary evaluate the immunomodulatory effects of ATO on Spike S glycoprotein (S protein)-stimulated freshly isolated human Peripheral Blood Mononuclear Cells (hPBMCs). The production of cytokines by freshly isolated hPBMCs after stimulation with different concentrations of Spike S glycoprotein from SARS-CoV-2 was evaluated, along with the immunomodulatory effect of 2.5 µM of ATO (Arscimed) in basal condition and after stimulations.

Study variable and end points: IL-6, TNF-α, IL-8, IL-1β production in supernatant after 24h of culture.

### Methods:

### PBMCs isolation

Under the authorization from the "Ministère de la recherche" n°DC-2018-3187 to manage human biological samples, blood bags from 3 different donors were obtained from "EFS Hauts-de-France-Normandie" (PLER-UPR/2018/082).

15 ml of density gradient medium (Ficoll) were added in 50 ml Falcon tubes. For each donor, blood was diluted to 1:2 in PBS and was added carefully above the density gradient medium. Falcon tubes were centrifuged during 20 minutes at 1500 rpm (Revolutions Per Minutes) at Room Temperature (RT) without brakes to avoid destabilizing the density gradient. PBMCs formed a circular layer in the serum and were harvested carefully by aspiration with a Pasteur pipette and added into a fresh 50 ml canonical tube. PBMCs were washed 2 times in PBS in a final volume of 50 ml with centrifugation step of 10 min at 1000 rpm at RT (with brakes on).

The supernatant was discarded and 10 ml of complete medium (RPMI1640 supplemented with 10 % FBS and 1% Penicillin/Streptomycin) were added. Cells were counted and resuspended in complete medium at 2x10⁶ cells/ml.

### Formulation

### S Spike glycoprotein

S Spike protein (Sinobiological, batch No. 40589-V0881) was resuspended in buffer (Ultrapure water) according to the manufacturer recommendation. Then, the stock solution was diluted adequately in complete medium and added in corresponding wells to reach final desired concentrations (*i.e*., 0.1, 1, 10, 50 and 100 nM) (Dorsch et al., 2009).

**Positive control**

LPS (standard TLR-4 agonist - Sigma) was resuspended in PBS as a stock solution of 1mg/ml. Then, stock solution was diluted adequately in complete medium and added in corresponding wells to reach a final concentration of 1pg/ml of LPS.

### Vehicule and buffer

The vehicle (PBS) is supplied "ready to use" and was diluted in complete medium in the same manner as LPS and served as a negative control of LPS.

Buffer (Ultrapure water) was diluted in complete medium in the same manner as the 100nM S Spike protein and will serve as a negative control of S Spike protein.

### Assay procedure / cells treatment

The *in vitro* procedure was performed in triplicate in a total volume of 200 µl with 2x10⁵ cells per well in a 96 wells plate. To obtain this concentration of cells, 50 µl of cell suspension (previously prepared at 4x10⁶ cells/ml) were added into wells. Then, 50 µl of the stimulation (i.e. S Spike protein, LPS, buffer or vehicle) prepared 4 times concentrated were added. 50 µl of ATP prepared 4 times concentrated or complete medium were added. Finally, 50 µl of complete medium were added to achieve final concentration (see also Table 3).

### Study design

**Table 3: Study design (stimulation with Spike protein)**

| **Group** | **Stimulation** | **Treatment** | **Number of wells** |
|---|---|---|---|
| 1 | Vehicle | - | 3 wells |
| 2 | Buffer (negative control) | - | 3 wells |
| 3 | Buffer (negative control) | ATO (2.5 µM) | 3 wells |
| 4 | S protein 0.1 nM | - | 3 wells |
| 5 | S protein 0.1 nM | ATO (2.5 µM) | 3 wells |
| 6 | S protein 1 nM | - | 3 wells |
| 7 | S protein 1 nM | ATO (2.5 µM) | 3 wells |
| 8 | S protein 10 nM | - | 3 wells |
| 9 | S protein 10 nM | ATO (2.5 µM) | 3 wells |
| 10 | S protein 50 nM | - | 3 wells |
| 11 | S protein 50 nM | ATO (2.5 µM) | 3 wells |
| 12 | S protein 100 nM | - | 3 wells |
| 13 | S protein 100 nM | ATO (2.5 µM) | 3 wells |
| 14 | LPS 1 µg/ml | - | 3 wells |
| 15 | LPS 1 µg/ml | ATO (2.5 µM) | 3 wells |
| 16 | PBS (negative control of LPS) | - | 3 wells |
| 17 | PBS (negative control of LPS) | ATO (2.5 µM) | 3 wells |

Plate was then incubated at 37°C, 5% CO₂ during 24h.

Twenty-four hours following incubation with items, each well content was harvested in Eppendorf tubes and centrifuged at 2000 rpm for 8 minutes. Supernatants were collected and stored at - 70°C for cytokines analyses, while cell pellets were stored at -70°C for further optional analysis.

### Cytokines analysis

Cytokines (*i.e*. IL-6, TNFα, IL-8, IL-1β) were quantified by Multiplex according to manufacturer's instructions (Life Technologies). The reading was performed on MagPix instruments (Luminex). IL-6 and IL-8 were re-assessed by ELISA as samples were above the limit of detection. Samples were diluted at 1:200 for IL-6 and 1:100 for IL-8 and read on plate reader (Multiskan FC, Thermo Scientific).

### Statistical test

One-way Anova was performed to compare groups for cytokines production. An unpaired t test was performed to compare the relative production of cytokines at the higher dose of S Spike protein with or without ATO.

The statistical significance, p value, of the results is denoted as *p<0.05, **p<0.01 and ***p<0.001.

### Results

### • TNFα analysis

Concentration of TNFα from the 3 donors in basal conditions and after treatments with Buffer, S Spike protein at different concentrations (i. e., 0.1, 1, 10, 50 and 100 nM) or LPS (positive control of stimulation) in presence or absence of 2.5 µM of ATO was assessed by Multiplex. The limit of detection of TNFα as indicated by the Multiplex manufacturer was 6.35 pg/ml and samples in which TNFα was not detected were attributed this value.

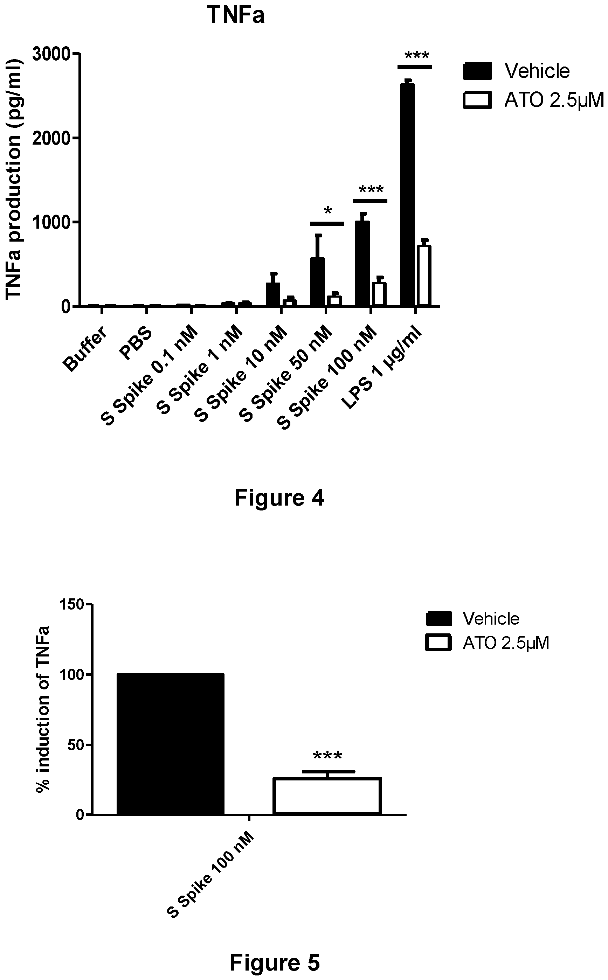

TNFα was not detected in basal condition and after treatment with buffer with or without 2.5µM of ATO for the 3 donors. Stimulation with S Spike protein dose dependently increased the production of TNFα with 12.56 ± 6.09 pg/ml at 0.1 nM, 30.35 ± 27.56 pg/ml at 1 nM, 263.39 ± 221.80 pg/ml at 10 nM, 565.75 ±481.71 pg/ml at 50 nM and 999.63 ± 177.34 pg/ml at 100 nM. The addition of ATO at 2.5 µM significantly inhibited the production of TNFα, especially at the higher doses (S Spike protein 50 nM = 565.75 ± 481.71 vs S Spike protein 50 nM + ATO 2.5 µM = 119.75 ± 66.49; S spike 100 nM = 999.63 ± 177.37 vs S Spike protein 100 nM + ATO 2.5 µM = 271.05 ± 128.14; p<0.05 and p<0.001, respectively). The positive control LPS induced a strong inflammatory response with a large production of TNFα which was inhibited by the addition of ATO (LPS 1 µg/ml= 20631.40 ± 93.94 vs LPS 1 µg/ml+ ATO 2.5µM = 710.82 ± 132.97; p<0.001). Mean concentrations of TNFα are presented in Table 4 and Figure 4.

**Table 4: Mean production of TNFα for the 3 donors (pg/ml) after stimulation with the different test items with or without ATO**

| | Mean production of TNFα (pg/ml) | SD |
|---|---|---|
| Vehicle | 6.35 | 0.00 |
| Buffer | 6.91 | 0.97 |
| Buffer + ATO 2,5µM | 6.81 | 0.22 |
| S Spike 0,1 nM | 12.56 | 6.09 |
| S Spike 0,1 nM + ATO 2,5 µM | 9.61 | 2.87 |
| S Spike 1 nM | 30.35 | 27.56 |
| S Spike 1 nM + ATO 2,5 µM | 30.37 | 41.31 |
| S Spike 10 nM | 263.39 | 221.80 |
| S Spike 10 nM + ATO 2,5 µM | 63.97 | 75.39 |
| S Spike 50 nM | 565.75 | 481.71 |
| S Spike 50 nM + ATO 2,5 µM | 119.75 * | 66.49 |
| S Spike 100 nM | 999.63 | 177.34 |
| S Spike 100 nM + ATO 2,5 µM | 271.05 *** | 128.14 |
| LPS 1µg/ml | 2631.40 | 93.94 |
| LPS 1µg/ml + ATO 2,5 µM | 710.82 *** | 132.97 |
| PBS | 6.35 | 0.00 |
| PBS + ATO 2,5 µM | 6.35 | 0.00 |

Noteworthy, the donor 2 was less responsive to the stimulation by the S spike protein and the production of TNFα was only increased at the dose of 100 nM of S spike protein (not shown). For this donor, at this concentration of stimulation, ATO 2.5 µM successfully inhibited the production of TNFα.

To reduce the variability between donor in response to 100 nM of S Spike protein, results were normalized as 100% for each donor and compared to results obtain with the addition of ATO. Such results are presented in Table 5 and Figure 5. At this dose of protein S Spike, the ATO 2.5 µM inhibited up to 64% the production of TNFα.

**Table 5: Relative production of TNFα for the 3 donors (%) after stimulation with 100 nM of S Spike protein**

| | Donor 1 | Donor 2 | Donor 3 | **Mean** | **SD** |
|---|---|---|---|---|---|
| S Spike 100 nM | 100% | 100% | 100% | **100%** | **0%** |
| S Spike 100 nM + ATO 2,5 µM | 35% | 25% | 19% | **26%** | **8%** |

### • IL-1β analysis

Concentration of IL-1β from the 3 donors in basal conditions and after treatments with Buffer, S Spike protein at different concentrations (i. e. 0.1, 1, 10, 50 and 100 nM) or LPS (positive control of stimulation) in presence or absence of 2.5 µM of ATO was assessed by Multiplex. The limit of detection of IL-1β as indicated by the Multiplex manufacturer was 2.36 pg/ml and samples in which IL-1β was not detected were attributed this value.

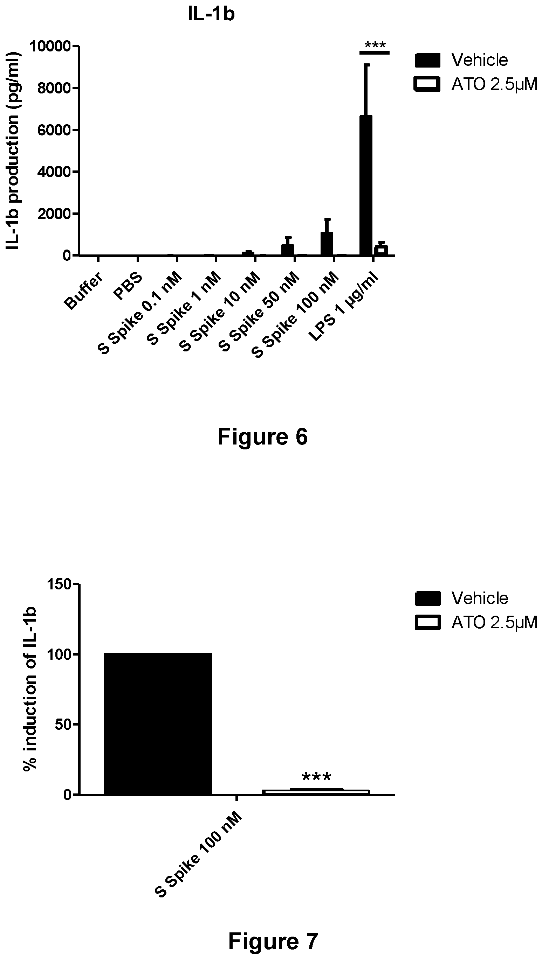

IL-1β was not detected in basal condition and after treatment with buffer with or without 2.5µM of ATO for the 3 donors. Stimulation with S Spike protein dose dependently increased the production of IL-1β with 8.10 ± 4.84 pg/ml at 0.1 nM, 8.46 ±5.76 pg/ml at 1 nM, 122.65 ± 111.66 pg/ml at 10 nM, 475.40 ±679.07 pg/ml at 50 nM and 1050.73 ± 1170.09 pg/ml at 100 nM. The addition of ATO at 2.5 µM non-significantly inhibited the production of IL-1β. The positive control LPS induced a strong inflammatory response with a large production of IL-1β which was inhibited by the addition of ATO (LPS 1 µg/ml= 6634.00 ± 4280.22 vs LPS 1 µg/ml+ ATO 2.5µM = 360.75 ± 484.13; p<0.001). Mean concentrations of IL1β are presented in Table 6 and Figure 6.

**Table 6: Mean production of IL-1β for the 3 donors (pg/ml) after stimulation with the different test items with or without ATO**

| | Mean production of IL-1β (pg/ml) | SD |
|---|---|---|
| Vehicle | 2.36 | 0.00 |
| Buffer | 2.97 | 1.07 |
| Buffer + ATO 2,5µM | 2.59 | 0.40 |
| S Spike 0,1 Nm | 8.10 | 4.84 |
| S Spike 0,1 nM + ATO 2,5 µM | 2.47 | 0.20 |
| S Spike 1 nM | 8.46 | 5.76 |
| S Spike 1 nM + ATO 2,5 µM | 4.73 | 3.80 |
| S Spike 10 nM | 122.65 | 111.66 |
| S Spike 10 nM + ATO 2,5 µM | 5.85 | 5.89 |
| S Spike 50 nM | 475.40 | 679.07 |
| S Spike 50 nM + ATO 2,5 µM | 11.65 | 4.34 |
| S Spike 100 nM | 1050.73 | 1170.09 |
| S Spike 100 nM + ATO 2,5 µM | 15.66 | 6.44 |
| LPS 1µg/ml | 6634.00 | 4280.22 |
| LPS 1µg/ml + ATO 2,5 µM | 360.75 *** | 484.13 |
| PBS | 2.48 | 0.10 |
| PBS + ATO 2,5 µM | 2.48 | 0.10 |

Noteworthy, the donor 2 was also less responsive to the stimulation by the S spike protein and the production of IL-1β was only increased at the dose of 100 nM of S spike protein (not shown). For this donor, the addition of 2.5 µM of ATO 2.5 µM successfully inhibited the production of IL-1β induced by the 100nM of S Spike protein.

To reduce the variability between donor in response to 100 nM of S Spike protein, results were normalized as 100% for each donor and compared to results obtain with the addition of ATO. Such results are presented in Table 7 and Figure 7. At this dose of protein S Spike, the ATO 2.5 µM inhibited up to 97% the production of IL-1β.

**Table 7: Relative production of IL-1β for the 3 donors (%) after stimulation with 100 nM of S Spike protein**

| | Donor 1 | Donor 2 | Donor 3 | **Mean** | **SD** |
|---|---|---|---|---|---|
| S Spike 100 nM | 100% | 100% | 100% | **100%** | **0%** |
| S Spike 100 nM + ATO 2,5 µM | 1% | 3% | 4% | **3%** | **1%** |

### • IL-6 analysis

Concentration of IL-6 from the 3 donors in basal conditions and after treatments with Buffer, S Spike protein at different concentrations (i. e. 0.1, 1, 10, 50 and 100 nM) or LPS (positive control of stimulation) in presence or absence of 2.5 µM of ATO was assessed by Multiplex and ELISA. The limit of detection of IL-6 as indicated by the Multiplex manufacturer was 8.06 pg/ml and samples in which IL-6 was not detected were attributed this value.

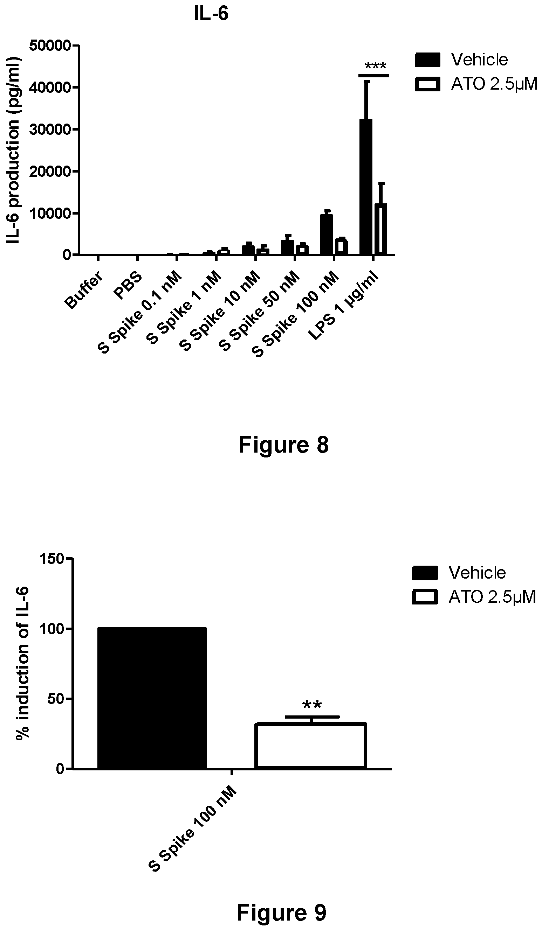

IL-6 was not significantly modulated in basal condition and after treatment with buffer with or without 2.5µM of ATO for the 3 donors. Stimulation with S Spike protein dose-dependently increased the production of IL-6 with 33.14 ± 29.37 pg/ml at 0.1 nM, 412.51 ± 538.10 pg/ml at 1 nM, 1887.67 ± 1586.58 pg/ml at 10 nM, 3277.30 ± 2416.03 pg/ml at 50 nM and 9480.44 ± 2024.54 pg/ml at 100 nM. The addition of ATO at 2.5 µM inhibited the production of IL-6, however, these results were not significant. The positive control LPS induced a strong inflammatory response with a large production of IL-6 which was inhibited by the addition of ATO (LPS 1 µg/ml= 32149.78 ± 16172.41 vs LPS 1 µg/ml+ ATO 2.5µM = 12062.00 ± 8715.29; p<0.001). Mean concentrations of IL-6 are presented in Table 8 and Figure 8.

**Table 8: Mean production of IL-6 for the 3 donors (pg/ml) after stimulation with the different test items with or without ATO.**

| | Mean production of IL-6 (pg/ml) | SD |
|---|---|---|
| Vehicle | 8.06 | 0.00 |
| Buffer | 20.68 | 12.64 |
| Buffer + ATO 2,5µM | 12.73 | 7.25 |
| S Spike 0,1 nM | 33.14 | 29.37 |
| S Spike 0,1 nM + ATO 2,5 µM | 80.54 | 84.98 |
| S Spike 1 nM | 412.51 | 538.10 |
| S Spike 1 nM + ATO 2,5 µM | 811.12 | 1380.91 |
| S Spike 10 nM | 1887.67 | 1586.58 |
| S Spike 10 nM + ATO 2,5 µM | 1202.18 | 1670.90 |
| S Spike 50 nM | 3277.30 | 2416.03 |
| S Spike 50 nM + ATO 2,5 µM | 1923.58 | 1222.20 |
| S Spike 100 nM | 9480.44 | 2024.54 |
| S Spike 100 nM + ATO 2,5 µM | 3146.11 | 1446.31 |
| LPS 1µg/ml | 32149.78 | 16172.41 |
| LPS 1µg/ml+ ATO 2,5 µM | 12062.00 *** | 8715.29 |
| PBS | 10.56 | 4.34 |
| PBS + ATO 2,5 µM | 8.89 | 1.4453 |

Noteworthy, the donor 2 was still less responsive to the stimulation by the S spike protein and the production of IL-6 was only increased from the dose of 50 nM of S spike protein (not shown). For this donor, ATO 2.5 µM successfully inhibited the production of IL-6 only at the 100 nM of S Spike stimulation.

To reduce the variability between donor in response to 100 nM of S Spike protein, results were normalized as 100% for each donor and compared to results obtain with the addition of ATO. Such results are presented in Table 9 and Figure 9. At this dose of protein S Spike, the ATO 2.5 µM inhibited up to 68% the production of IL-6.

**Table 9: Relative production of IL-6 for the 3 donors (%) after stimulation with 100 nM of S Spike protein**

| | Donor 1 | Donor 2 | Donor 3 | **Mean** | **SD** |
|---|---|---|---|---|---|
| S Spike 100 nM | 100% | 100% | 100% | **100%** | **0%** |
| S Spike 100 nM + ATO 2,5 µM | 42% | 28% | 27% | **32%** | **8%** |

### • IL-8 analysis

Concentration of IL-8 from the 3 donors in basal conditions and after treatments with Buffer, S Spike protein at different concentrations (i. e. 0.1, 1, 10, 50 and 100 nM) or LPS (positive control of stimulation) in presence or absence of 2.5 µM of ATO was assessed by Multiplex and ELISA. The limit of detection of IL-8 as indicated by the Multiplex manufacturer was 390.75 pg/ml and samples in which IL-8 was not detected were attributed this value.

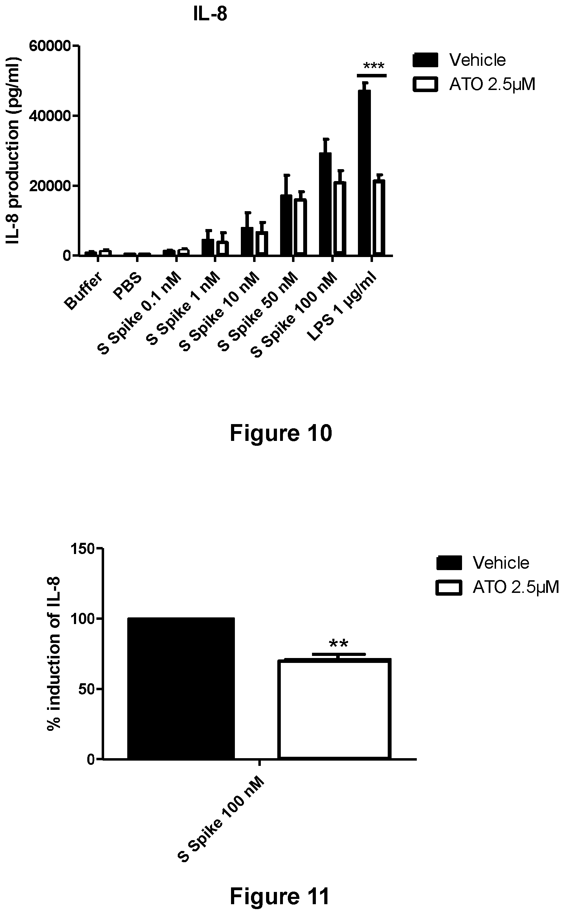

IL-8 was not significantly modulated in basal condition and after treatment with buffer with or without 2.5µM of ATO for the 3 donors. Stimulation with S Spike protein dose dependently increased the production of IL-8 with 1172.33 ± 869.56 pg/ml at 0.1 nM, 4352.08 ± 4855.27 pg/ml at 1 nM, 7818.33 ± 7711.01 pg/ml at 10 nM, 17097.89 ± 10137.93 pg/ml at 50 nM and 29110.22 ± 7231.32 pg/ml at 100 nM. The addition of ATO at 2.5 µM did not significantly modulated the production of IL-8 and only a slight decrease was observed with the higher dose of S Spike protein (100 nM of S Spike protein = 29110.22 ± 7231.32 pg/ml vs 100 nM of S Spike protein + 2.5 µM ATO = 20839.22 ± 6016.30). The positive control LPS induced a strong inflammatory response with a large production of IL-8 which was inhibited by the addition of ATO (LPS 1 µg/ml = 47042.11 ± 4166.78 vs LPS 1 µg/ml+ ATO 2.5µM = 21374.11 ± 3003.19; p<0.001). Mean concentrations of IL-8 are presented in Table 10 and Figure 10.

**Table 10: Mean production of IL-8 for the 3 donors (pg/ml) after stimulation with the different test items with or without ATO**

| | Mean production of IL-8 (pg/ml) | SD |
|---|---|---|
| Vehicle | 502.64 | 41.20 |
| Buffer | 791.47 | 568.47 |
| Buffer + ATO 2,5µM | 1172.33 | 869.56 |
| S Spike 0,1 nM | 1225.81 | 476.94 |
| S Spike 0,1 nM + ATO 2,5 µM | 1348.56 | 1006.03 |
| S Spike 1 nM | 4352.08 | 4855.27 |
| S Spike 1 nM + ATO 2,5 µM | 3635.50 | 4982.18 |
| S Spike 10 nM | 7818.33 | 7711.01 |
| S Spike 10 nM + ATO 2,5 µM | 6551.00 | 5114.77 |
| S Spike 50 nM | 17097.89 | 10137.93 |
| S Spike 50 nM + ATO 2,5 µM | 15540.61 | 4697.32 |
| S Spike 100 nM | 29110.22 | 7231.32 |
| S Spike 100 nM + ATO 2,5 µM | 20839.22 | 6016.30 |
| LPS 1µg/ml | 47042.11 | 4166.78 |
| LPS 1 µg/ml+ ATO 2,5 µM | 21374.11 *** | 3003.19 |
| PBS | 430.94 | 34.89 |
| PBS + ATO 2,5 µM | 445.31 | 58.80 |

Noteworthy, the donor 2 was still less responsive to the stimulation by the S spike protein and the production of IL-8 was only increased from the dose of 50 nM of S spike protein (not shown). For this donor, ATO 2.5 µM successfully inhibited the production of IL-8 only at the 100 nM of S Spike stimulation.

To reduce the variability between donor in response to 100 nM of S Spike protein, results were normalized as 100% for each donor and compared to results obtain with the addition of ATO. Such results are presented in Table 11 and Figure 11. At this dose of protein S Spike, the ATO 2.5 µM inhibited up to 29% the production of IL-8.

**Table 11: Relative production of IL-8 for the 3 donors (%) after stimulation with 100 nM of S Spike protein**

| | Donor 1 | Donor 2 | Donor 3 | **Mean** | **SD** |
|---|---|---|---|---|---|
| S Spike 100 nM | 100% | 100% | 100% | **100%** | **0%** |
| S Spike 100 nM + ATO 2,5 µM | 72% | 64% | 77% | **71%** | **7%** |

**Conclusion:** Under study conditions, S Spike protein triggers an immunological response in human PBMC. Indeed, S Spike protein dose dependently triggers the production of TNFα, IL-1β, IL-6 and IL-8. Interestingly, this response seemed to be donor dependent as one donor (donor #2) appeared to be less responsive to the S Spike stimulation. In comparison, the 3 donors were well responsive to the TLR-4 stimulation (LPS 1 µg/ml), which induced a strong inflammatory response. We can hypothesize that the donor #2 possess less receptors implicated in the immunological response to the S Spike protein. At the higher tested concentration of S Spike protein (100 nM), which induced an immunological response on all the donors, the addition of ATO significantly inhibited the production of TNFα, IL-1β, IL-6 and IL-8.

### Example 3: Treatment of a patient suspected or confirmed to have an infection by a SARSr-CoV2

A patient suspected or confirmed to have an infection by a SARSr-CoV2 is treated as follows:
a/ At first symptoms : use any standard of care for RNA type viral infections to start decreasing the viral load, such as, e.g., Hydroxychloroquine (200-600 mg/day), or Dexamethasone with azythromycine (200 mg/day) and Zn+ (15 mg/day),
b/ Upon worsening of clinical signs (such as slight respiratory impairment): if possible, immediately test for "Coronavirus" cytokines - or a series of known virally induced cytokines - to be able (e.g., as soon as some levels of proinflammatory cytokines are up by at least three times their normal circulatory levels) to decide to start a new specific treatment for inhibiting the possible - or irrupting - cytokine storm, as follows:
   c/ Deliver to the patient Arsenic trioxide as an IV, or oral (when available), or aerosol spray-administered drug, or oral forms, using O;075 to 0.30 mg/day of As salt, the preferred dosing being 0.15 mg/day of As₂O₃, in possible further association with synergic ingredients such as metal ions, (e.g., copper salts) or other drugs with expected beneficial effects on levels of proinflammatory cytokines, such as dexamethasone or thalidomide derivatives.

### Example 4: IL-1β stimulated by the Protein PX is inhibited by arsenic salts (arsenic trioxide)

**Objective:** The objective of this study was to determine the immunomodulatory effects of arsenic trioxide (ATO) on fresh human Peripheral Blood Mononuclear Cells (hPBMCs) after stimulation by the HERV W Env protein (also called Protein PX).

**Methods:** PBMCs were prepared as described in example 1 above and stimulated with Protein PX in presence or absence of ATO in different conditions. LPS was used as a positive control and dexamethasone as a control of inhibition of cytokine production. Twenty-four hours after stimulation, supernatants were harvested for cytokines analysis.

Protein PX was supplied at 930 µg/mlin Buffer (50 mM 2-(N-morpholino)ethanesulfonic acid (MES), 10 mM Dithiotheitol (DTT), 10 % glycerol, pH = 6.0). Protein PXwas dissolved in complete medium at 1:100 (9.3 µg/ml).

LPS (TLR-4 agonist) was resuspended in PBS as a stock solution at 1 mg/ml. Then, stock solution was diluted adequately in complete medium and added in corresponding wells to reach a final concentration of 1 µg/mlof LPS.

The vehicle was complete medium.

Buffer was diluted at 1:100 in complete medium and served as a negative control of Protein PX.

PBS was diluted adequately in complete medium and served as a negative control of ATO and CuCl₂.

Ethanol was diluted adequately in complete medium and served as a negative control of Dexamethasone.

### Study design

The *in vitro* procedure was performed in triplicate in a total volume of 200 µl with 2x10⁵ cells per well in a 96 wells plate. To obtain this concentration of cells, 50 µl of cell suspension (previously prepared at 4x10⁶ cells/ml) were added into wells. Then, 50 µl of the different treatments previously prepared 4 times concentrated (i.e. Protein PX, ATO, CuCl2, LPS, dexamethasone) or the vehicles were added in order to obtain the final desired concentration of each item (see also Table 12).

**Table 12: study design (Protein PX)**

| **Group** | **Stimulation** | **Treatments** | **Number of wells** |
|---|---|---|---|
| 1 | Vehicle | Vehicle | 3 wells |
| 2 | LPS (1 µg/ml) | Vehicle | 3 wells |
| 3 | | Dexamethasone (1µM) | 3 wells |
| 4 | Protein PX (1:100) | Vehicle | 3 wells |
| 5 | | ATO (2,5µM) | 3 wells |
| 6 | | CuCl₂ (1,25 µM) | 3 wells |
| 7 | | ATO (2,5 µM) + CuCl₂ (1,25 µM) | 3 wells |
| 8 | | Dexamethasone (1µM) | 3 wells |

Twenty-four hours following incubation with the controls and test items, each well content was harvested in Eppendorf tubes and centrifuged at 2000 rpm for 8 minutes. Supernatants were collected and stored at -70°C for cytokines analysis, while cell pellets were stored at -70°C for further optional analysis (upon sponsor request).

Human IFNα, TNFα, IL-1β, IL-8, IL-12, GM-CSF, IFNγ, IL-6, IL-2, IL-4, S100 (A8 and A9) were quantified by Multiplex according to the manufacturer's instructions (Life Technologies). The reading were performed on MagPix instruments (Luminex).

### Results:

A strong stimulation of cytokine production by the PX protein was observed for IL-1β, especially, and to a lesser extent, for IL6, TNFα, IL10, and IL8.

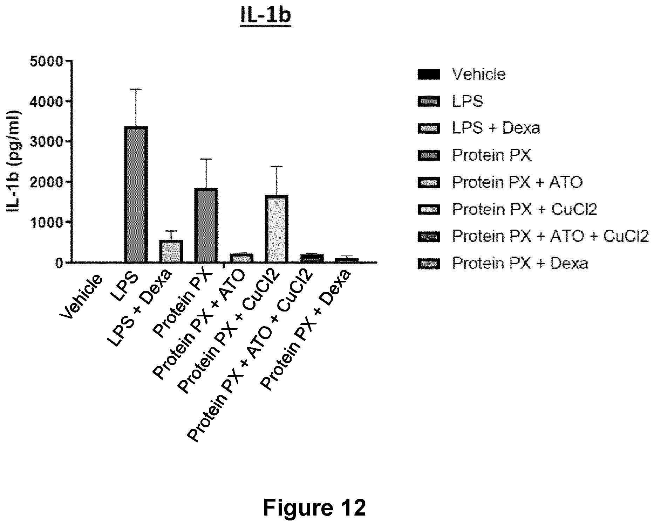

As shown in Figure 12, arsenic trioxide (2.5 µM) strongly inhibited IL-1β production despite stimulation by protein PX, showing a very strong anti-inflammatory activity.

**Conclusion:** After stimulation by protein PX, ATO strongly inhibited the production of IL-1β, as well as the production of IL-6.

This illustrates the interest of ATO in the context of therapy for multiple sclerosis and other inflammatory "cytokine storm" diseases involving IL-1β. In this context, arsenic could advantageously be administered in combination with inhibitors specific for cytokines other than IL-1β and IL-6 and possibly involved in the proinflammatory and/or degenerative pathological process.

### REFERENCES

Akdis et al. 2016 J Allergy Clin Immunol., Volume 138, Number 4, 984-1010. Charley, B ; Bull. Acad. Vét. France - 2003 - Tome 156 pp 31-36 - Supplement to N° 3
Rodero M.P. and Crow Y.J., 2016. Type 1 interferon- mediated monogenic autoinflammation: The type 1 interferonopathies, a conceptual overview; J. Exp. Med. 213, 2527-2538
Dosch et al., SARS coronavirus spike protein-induced innate immune response occurs via activation of the NF-Kb pathway in human monocyte macrophages in vitro. Virus research. 2009; 142: 19-27.
Hamidou et al. 2021 Safety and efficacy of low-dose intravenous arsenic trioxide in systemic lupus erythematosus: an open-label phase IIa trial (Lupsenic). Arthritis Research & Therapy
Huang, C. et al. Clinical features of patients infected with 2019 novel coronavirus in Wuhan, China. Lancet 395, 497-506 (2020).
Imai Y, et al. 2008. Identification of oxidative stress and Toll-like receptor 4 signaling as a key pathway of acute lung injury. Cell 133:235-249.
Levy MM, et al. 2003. 2001 SCCM/ESICM/ACCP/ATS/SIS International Sepsis Definitions Conference. Crit. Care Med. 31:1250 -1256.
Michel O, et al. 2003. Systemic responsiveness to lipopolysaccharide and polymorphisms in the Toll-like receptor 4 gene in human beings. J. Allergy Clin. Immunol. 112:923-929.
Mockenhaupt FP, et al. 2006. Toll-like receptor (TLR) polymorphisms in African children: common TLR-4 variants predispose to severe malaria. Proc. Natl. Acad. Sci. U. S. A. 103:177-182.
Mockenhaupt FP, et al. 2006. Common polymorphisms of Toll-like receptors 4 and 9 are associated with the clinical manifestation of malaria during pregnancy. J. Infect. Dis. 194:184-188.
Sun L, et al. 2011. New concepts of IL-10-induced lung fibrosis: fibrocyte recruitment andM2activation in a CCL2/CCR2 axis. Am. J. Physiol. Lung Cell. Mol. Physiol. 300:L341-L353.
Wurfel MM, et al. 2005. Identification of high and low responders to lipopolysaccharide in normal subjects: an unbiased approach to identify modulators of innate immunity. J. Immunol. 175:2570 -2578.

## Claims

1. A composition comprising an arsenic compound selected from the group consisting of As₂O₃, AsI₃, As₂O₅, As₄O₆, As₂S₂, As₂S₃, As₂S₅, As₄S₄ and mixtures thereof, for use for treating a sepsis syndrome.

2. The composition for use according to claim 1, wherein the arsenic compound is As₂O₃.

3. The composition for use according to claim 1, wherein the arsenic compound is a mixture of As₂O₃and As₂O₅.

4. The composition for use according to any one of claims 1 to 3, wherein As₂O₃ is administered to a subject in need thereof at a daily dose of 0.05 mg/kg to 0.5 mg/kg of bodyweight, preferably at a daily dose of 0.075 mg/kg to 0.30 mg/kg of bodyweight.

5. The composition for use according to claim 4, wherein As₂O₃ is administered to a subject in need thereof at a daily dose of 0.10 mg/kg to 0.30 mg/kg of bodyweight, preferably 0.10 mg/kg to 0.20 mg/kg of bodyweight.

6. The composition for use according to any one of claims 1 to 5, wherein the composition further comprises a metal ion selected from the group consisting of Cu²⁺, Au²⁺, Fe²⁺, Zn²⁺, Mn²⁺, Mg²⁺ and mixtures thereof, preferably Cu²⁺.

7. The composition for use according to any one of claims 1 to 6, wherein the arsenic compound is administered via nanoparticles.

8. The composition for use according to any one of claims 1 to 7, wherein the arsenic compound is administered orally, topically or as an aerosol spray.

9. The composition for use according to any one of claims 1 to 7, wherein the arsenic compound is administered intravenously.

10. The composition for use according to claim 9, wherein the arsenic compound is administered intravenously at a daily dose of 0.075 mg/kg to 0.30 mg/kg, preferably 0.15 mg/kg.

11. The composition for use according to any one of claims 1 to 10, wherein the arsenic compound is administered with corticosteroids or monoclonal antibodies directed towards any relevant proteic component of the immune system.

12. The composition for use according to any one of claims 1 to 11, wherein the arsenic compounds is administered in combination with dexamethasone or in combination with thalidomide derivatives.

13. The composition for use according to any one of claims 1 to 12, wherein the composition reduces IFNα, TNFα, IL-6, IL-1β, IL-8, GM-CSF, IL17, IL23 and/or IL-10 production by peripheral blood mononuclear cells (PBMCs), preferably the composition reduces TNFα, IL-1β, IL-6, and IL-8 production by peripheral blood mononuclear cells (PBMCs).

14. The composition for use according to any one of claims 1 to 13 for treating a sepsis syndrome caused by a viral, bacterial, and/or fungal pulmonary infections.

15. The composition for use according to claim 14, for treating bacterial sepsis.
